# EUROPÄISCHE PATENTSCHRIFT

(11) **EP 3 706 659 B1**
(45) Veröffentlichungstag und Bekanntmachung des Hinweises auf die Patenterteilung: **28.09.2022**
(21) Anmeldenummer: 18811446.6
(22) Anmeldetag: 07.11.2018
(51) Int. Cl.: A61B 90/14, A61N 5/10, A61B 6/04

(54) **RADIO-ONKOLOGISCHE KOPFPOSITIONIERUNGSEINRICHTUNG UND RADIO-ONKOLOGISCHE PATIENTENPOSITIONIERUNGSVORRICHTUNG**
RADIO-ONCOLOGICAL HEAD-POSITIONING EQUIPMENT AND RADIO-ONCOLOGICAL PATIENT-POSITIONING DEVICE
SYSTÈME RADIO-ONCOLOGIQUE DE POSITIONNEMENT DE LA TÊTE ET DISPOSITIF RADIO-ONCOLOGIQUE DE POSITIONNEMENT DU PATIENT

(30) Priorität: 07.11.2017 DE 102017126025
(43) Veröffentlichungstag der Anmeldung: 16.09.2020
(73) Patentinhaber: Landratsamt Ortenaukreis Körperschaft des öffentlichen Rechts, 77652 Offenburg (DE)
(72) Erfinder: SCHUMANN, Dirk, 77654 Offenburg (DE)
(74) Vertreter: Wittmann, Günther
(86) Internationale Anmeldenummer: PCT/EP2018/080505
(87) Internationale Veröffentlichungsnummer: WO 2019/092039

(56) Entgegenhaltungen:
- WO-A2-00/27331
- US-A1- 2009 308 400

## Beschreibung

Die vorliegende Erfindung betrifft eine verbesserte radio-onkologische Kopfpositionierungseinrichtung und eine verbesserte radio-onkologische Patientenpositionierungsvorrichtung, die den Patienten reproduzierbarer und mit weniger Spiel in einer radio-onkologischen Vorrichtung positioniert.

### Stand der Technik

Um vorbestimmtes Gewebe eines Patienten während einer radio-onkologischen Behandlung zu bestrahlenden, werden Patientenpositionierungsvorrichtungen verwendet, um den Patienten und insbesondere das zu bestrahlende Gewebe reproduzierbar gegenüber der Strahlungsquelle zu positionieren. Dadurch kann sichergestellt werden, dass einerseits das, beispielsweise von einem Tumor, befallene Gewebe bestrahlt wird, aber kein gesundes Gewebe geschädigt wird.

Insbesondere um befallenes Gewebe im Kopf zu behandeln, werden so genannte Hinterkopfmasken, Gesichtsmasken sowie Ganzschädelmasken verwendet werden. Ferner sind Halsmasken und Schultermasken bekannt.

Mit Lagerungshilfen des Standes der Technik können in der Radio-Onkologie verschiedene Körperteile durch geeignete Vorrichtungen direkt auf dem Bestrahlungstisch oder durch anzubauende Vorrichtungen gelagert werden. Durch jeden Anbau von Vorrichtungen können Abweichungen im Bereich des Bestrahlungsfeldes und darüber hinaus entstehen, was zu unerwünschten Ungenauigkeiten führen kann. Dies hat zudem auch einen höheren zeitlichen Umfang sowie mehr Körperbelastungen für das Personal.

Die US 2009/0308400 A1 offenbart eine Vorrichtung zum Immobilisieren eines Patienten. Die Vorrichtung umfasst ein Paar von sich längs erstreckenden Seitenschienen mit einer Mehrzahl von in Längsrichtung angeordneten Öffnungen zum Aufnehmen eines Verriegelungsträgers, an den eine Fixierkomponente angebracht werden kann.

Die WO 00/27331 A2 betrifft eine Vorrichtung zum Halten der Position eines Patienten während einer medizinischen Prozedur, beispielsweise für PET-Bildgebung, für MRI-Bildgebung oder für radiotherapeutischen Prozeduren, in denen es wichtig ist, dass sich der Körper des Patienten an einer stationären Position befindet. Die Vorrichtung umfasst ein System zum Nachverfolgen der Bewegung eines bestimmten Körperteils, beispielsweise des Kopfes, einen Computer zum Empfangen von Signalen von dem nach Verfolgungssystem und ermitteln, ob eine unerwünschte Bewegung des Körpers aufgetreten ist. Falls eine unerwünschte Bewegung des Körpers aufgetreten ist, wird ein Korrektursignal ausgegeben, das mittels einer Mehrzahl von Motoren ein Repositionieren des Körperbereichs zum gewünschten Ort bewirkt. Dieses Überwachen und Repositionieren kann bei einer sehr hohen Frequenz stattfinden. Eine spezielle in Betracht gezogene Anwendung ist die Immobilisierung und Positionierung des Kopfes während Gehirnuntersuchungen.

Radio-onkologische Patientenpositionierungseinrichtungen des Standes der Technik können aus einer Mehrzahl von Komponenten modular zusammengestellt werden.

### Allgemeine Beschreibung der Erfindung

Die Erfindung stellt sich daher zur Aufgabe, eine radio-onkologische Kopfpositionierungseinrichtung und eine radio-onkologische Patientenpositionierungseinrichtung mit einer verbesserten Reproduzierbarkeit der Lagerung des Patienten zu schaffen.

Die Aufgabe der Erfindung wird durch eine radio-onkologische Patientenpositionierungsvorrichtung nach Anspruch 1 gelöst. Die abhängigen Ansprüche beanspruchen bevorzugte Ausführungsformen.

Eine erfindungsgemäße radio-onkologische Patientenpositionierungsvorrichtung umfasst einen Rumpfstützbereich, der dazu ausgebildet ist, den Rumpf eines Patienten zu stützen. Der Rumpfsützbereich umfasst erste Positionierungselemente, die dazu eingerichtet sind eine Kniepositionierungseinrichtung und/oder eine Beckenpositionierungseinrichtung lösbar anzuordnen. Der Rumpfstützbereich umfasst einen Schulterstützbereich, der zweite Positionierungselemente aufweist, die dazu eingerichtet sind, eine Halspositionierungseinrichtung und/oder eine Schulterpositionierungseinrichtung lösbar anzuordnen. Die Patientenpositionierungsvorrichtung umfasst ferner einen Kopfstützbereich, wobei der Kopfstützbereich mittels zumindest einem Gelenk schwenkbar am Schulterstützbereich oder an einer Basisplatte angeordnet ist, auf der der Rumpfstützbereich angeordnet ist, und wobei der Kopfstützbereich so eingerichtet ist, dass eine radio-onkologische Kopfpositionierungseinrichtung lösbar am Kopfstützbereich anbringbar ist, wobei an der Kopfpositionierungseinrichtung eine Hinterkopfmaske und/oder eine Gesichtsmaske anordenbar ist. Der Kopfstützbereich kann fest, d.h. im bestimmungsgemäßen Einsatz nicht lösbar, am Rumpfstützbereich angeordnet sein.

Dadurch wird der Patient präzise und reproduzierbar während der gesamten Bestrahlungsperiode positioniert. Die einzelnen Zusatzkomponenten können durch die Positionierungselemente jeweils exakt am Anfang der Behandlungsperiode auf den Patienten adaptiert werden und deren Stellung auf der Patientenpositionierungsvorrichtung wird bis zum Ende der Behandlungsperiode beibehalten. Zudem reduziert sich der Zeitaufwand für die einzelnen Bestrahlungen, da die verschiedenen Bestrahlungsvorrichtungen direkt und ohne Umbau positioniert werden, sowie die körperliche Anstrengung des Personals durch leichte Patientenpositionierungsvorrichtung aus Carbon.

Der Rumpfstützbereich ist dazu ausgebildet, den Rumpf eines Patienten zu stützen. Der Kopfstützbereich ist dazu ausgebildet, den Kopf eines Patienten zu stützen. Der Kopfstützbereich ist fest am Rumpfstützbereich bzw. an der Basisplatte angeordnet und mittels zumindest eines Gelenkes (Scharniers) schwenkbar am Rumpfstützbereich angeordnet. Die radio-onkologische Kopfpositionierungseinrichtung ist am Kopfstützbereich lösbar angeordnet.

Dadurch, dass der Kopfstützbereich fest mittels zumindest eines Gelenkes schwenkbar am Rumpfstützbereich bzw. an der Basisplatte angeordnet ist, werden Toleranzen und das Spiel reduziert, wodurch die reproduzierbare Positionierung des Patienten auch über mehrere Behandlungszyklen gewährleistet ist. Die radio-onkologische Kopfpositionierungseinrichtung kann über Führungselemente und Führungszapfen spielfrei am Kopfstützbereich lösbar angebracht werden.

Der Kopfstützbereich kann eine Arretierungsvorrichtung aufweisen, die ermöglicht, den Kopfstützbereich unter unterschiedlichen Winkelstellungen gegenüber dem Rumpfstützbereich einstellbar abzustützen.

Der Rumpfstützbereich kann Positionierungselemente aufweisen, an denen eine Kniepositionierungseinrichtung lösbar anordenbar ist. Der Rumpfstützbereich kann Positionierungselemente aufweisen, an denen eine Beckenpositionierungseinrichtung lösbar anordenbar ist. Die Positionierungselemente können Ausnehmungen, beispielsweise halbkreisförmige

Ausnehmungen am Rand des Rumpfstützbereichs sein, an denen die Kniepositionierungseinrichtung und/oder die Beckenpositionierungseinrichtung lösbar angeordnet werden können.

Bei einer Ausführungsform kann der Rumpfstützbereich einen Schulterstützbereich aufweisen, der zweite Positionierungselemente für eine Halspositioniereinrichtung aufweist. Der Schulterstützbereich kann alternativ oder zusätzlich auch zweite Positionierungselemente für eine Schulterpositionierungseinrichtung aufweisen.

Die zweiten Positionierungseinrichtungen können zylinderförmige Vertiefungen für die Einlagen für Schulter und/oder Hals sein.

Die ersten Positionierungselemente können Positionierungselemente für Positioniersysteme, Knieschienen, Beckenstützeinrichtungen oder dergleichen sein.

Die Kopfpositionierungseinrichtung und/oder die Patientenpositionierungseinrichtung können zumindest teilweise aus Carbon hergestellt werden. Zum einen wird dadurch das Gewicht reduziert, so dass die Kopfpositionierungseinrichtung sowie die Patientenpositionierungseinrichtung leichter durch Personal gehandhabt werden. Ferner beeinflussen Carbonmaterialien in einem geringeren Maße die radio-onkologische Behandlung als Metallmaterialien.

Der Erfinder der vorliegenden Erfindung bezeichnet die erfindungsgemäße Patientenpositionierungsvorrichtung als Offenburger Multilagerungsboard aus Carbon. Damit können sämtliche Bestrahlungstechniken auf einer Patientenpositionierungsvorrichtung ausgeführt werden. Das Board ist komplett durchstrahlfähig, da es kein Metall aufweist.

Die Basisplatte kann sich unter dem Rumpfstützbereich und unter dem Kopfstützbereich zumindest teilweise erstrecken. Die Basisplatte kann sich unter dem gesamten Rumpfstützbereich und Kopfstützbereich erstrecken.

Eine radio-onkologische Kopfpositionierungseinrichtung, die zum Halten des Kopfes eines Patienten während einer radio-onkologischen Behandlung ausgebildet ist, umfasst eine Grundplatte, zumindest ein Stützelement, einen ersten U-förmigen Rahmen, einen zweiten U-förmigen Rahmen und zwei erste L-förmige Befestigungselemente. Die Grundplatte kann Koppelelemente zum Koppeln der radio-onkologischen Kopfpositionierungseinrichtung mit einer radio-onkologischen Patientenpositionierungseinrichtung aufweisen. Das zumindest eine Stützelement erstreckt sich von der Grundplatte. Der Hinterkopf des Patienten ist während der Behandlung in Richtung Grundplatte gerichtet. Das zumindest eine Stützelement erstreckt sich im wesentlichen neben dem Kopf des Patienten von der Grundplatte weg. Beispielsweise können vier säulenartige Stützelemente verwendet werden. Bei einer anderen Ausführungsform können zwei quaderförmige Stützelemente oder drei quaderförmige Stützelemente verwendet werden.

Der erste U-förmigen Rahmen ist an dem zumindest einen Stützelement angeordnet. Der erste U-förmigen Rahmen kann starr an dem zumindest einen Stützelement angeordnet sein. Der zweite U-förmigen Rahmen ist lösbar an dem ersten U-förmigen Rahmen angeordnet. Die zwei ersten L-förmigen Befestigungselemente sind am zweiten U-förmigen Rahmen angeordnet.

Zwischen dem ersten U-förmigen Rahmen und dem zweiten U-förmigen Rahmen kann eine Hinterkopfmaske angeordnet werden. An den zwei L-förmigen Befestigungselementen kann eine Gesichtsmaske angeordnet werden. Die erfindungsgemäße radio-onkologische Kopfpositionierungseinrichtung ermöglicht, dass die Gesichtsmaske und die Hinterkopfmaske stabil gelagert werden und so der Kopf des Patienten reproduzierbar auch über mehrere Behandlungen hinweg reproduzierbar an den zwei ersten L-förmigen Befestigungselementen gelagert wird. An der Grundplatte kann ein Nackenkissen angeordnet sein, um den Komfort des Patienten zu erhöhen.

Bei einer Ausführungsform sind zwei zweite L-förmige Befestigungselemente losbar angeordnet. Je ein zweites L-förmiges Befestigungselement ist an einem ersten L-förmigen Befestigungselement angeordnet. Bei einer bevorzugten Ausführungsform ist der längere Schenkel des zweiten L-förmigen Befestigungselementes über dem längeren Schenkel des ersten L-förmigen Befestigungselement angeordnet. Bei dieser Ausführungsform ist der kürzere Schenkel des zweiten L-förmigen Befestigungselementes über dem kürzeren Schenkel des ersten L-förmigen Befestigungselementes angeordnet.

Die ersten und zweiten L-förmigen Befestigungselemente sind an der Seite des zweiten U-förmigen Rahmens angeordnet, die der Grundplatte entgegengesetzt ist. Auch der zweite U-förmigen Rahmen ist an der Seite des ersten U-förmigen Rahmens angeordnet, die der Grundplatte entgegengesetzt ist. Die Schenkel der ersten und zweiten L-förmigen Befestigungselemente sind über den Schenkeln des zweiten U-förmigen Rahmens angeordnet. Die kürzeren Schenkel der zwei ersten L-förmigen Befestigungselemente können über dem mittleren Schenkel des zweiten U-förmigen Rahmens angeordnet sein. Die längeren Schenkel der zwei ersten L-förmigen Befestigungselemente können an den Schenkeln des zweiten U-förmigen Rahmens angeordnet sein, die sich von dem mittleren Schenkel (oder die beiden anderen Schenkel verbindenden Schenkel) des zweiten U-förmigen Rahmens erstrecken, angeordnet sein. Die beiden zweiten L-förmigen Befestigungselemente ermöglichen eine sichere Befestigung der Gesichtsmaske an den ersten L-förmigen Befestigungselementen.

Die radio-onkologische Kopfpositionierungseinrichtung kann bei einer Ausführungsform ein verschiebbares Klemmelement aufweisen, das in Längsrichtung des ersten U-förmigen Rahmens und in Längsrichtung des zweiten U-förmigen Rahmens verschiebbar ist. Als Längsrichtung wird diejenige Richtung bezeichnet, in welcher der erste U-förmigen Rahmen und der zweite U-förmigen Rahmen offen sind. Das Klemmelement drückt in seiner geschlossenen Stellung die zwei ersten L-förmigen Befestigungselemente gegen den zweiten U-förmigen Rahmen. In dieser Stellung wird eine Gesichtsmaske zwischen den zwei L-förmigen Befestigungselementen und dem zweiten U-förmigen Rahmen gehalten. In seiner offenen Stellung ist das Klemmelement in Längsrichtung vom offenen Ende des ersten U-förmigen Rahmens und des zweiten U-förmigen Rahmens betrachtet hinter den zwei L-förmigen Befestigungselementen angeordnet. Das Klemmelement ermöglicht eine einfache und schnelle Fixierung der ersten L-förmigen Befestigungselemente und der Gesichtsmaske. Es versteht sich, dass die Gesichtsmaske mittels Schrauben oder Bolzen, die in Öffnungen eingeführt werden, die sich durch die zweiten L-förmigen Befestigungselemente und ersten L-förmigen Befestigungselemente erstrecken, befestigt werden kann.

Die Erfindung betrifft auch eine radio-onkologische Kopfpositionierungseinrichtung, die zum Halten des Kopfes eines Patienten während einer radio-onkologischen Behandlung ausgebildet ist, die eine Grundplatte, zwei erste L-förmige Befestigungselemente und zwei zweite L-förmige Befestigungselemente aufweist. Die Grundplatte ist zum Koppeln mit einer radio-onkologischen Patientenpositionierungsvorrichtung ausgebildet, beispielsweise kann die Grundplatte Koppelelemente aufweisen. Die zwei ersten L-förmige Befestigungselemente sind an der Grundplatte angeordnet. Die ersten L-förmigen Befestigungselementen können lösbar oder fest angeordnet sein.

Die zweiten L-förmigen Befestigungselemente sind an den ersten L-förmigen Befestigungselementen lösbar angeordnet. Zwischen den ersten L-förmigen Befestigungselementen und den zweiten L-förmigen Befestigungselemente kann eine Gesichtsmaske angeordnet werden.

Die Grundplatte kann Aufnahmeelementes, beispielsweise Öffnungen, Ausnehmungen oder dergleichen, aufweisen, die zum Positionieren eines Nackenkissens ausgebildet sind.

Die radio-onkologische Kopfpositionierungseinrichtung kann ferner ein Klemmelement aufweisen, das in Längsrichtung der radio-onkologischen Kopfpositionierungseinrichtung verschiebbar ist. Das verschiebbare Klemmelement drückt in seiner geschlossenen Stellung die zwei ersten L-förmigen Befestigungselemente gegen die Grundplatte. In seiner offenen Stellung befindet sich das verschiebbare Klemmelement in Längsrichtung vom offenen Ende der zwei ersten L-förmigen Befestigungselemente betrachtet hinter den zwei ersten L-förmigen Befestigungselementen.

Bei beiden Ausführungsformen der Kopfpositionierungseinrichtung sind die zwei ersten L-förmigen Befestigungselemente und die zweite zweiten L-förmigen Befestigungselemente so angeordnet, dass sie in Draufsicht eine im wesentlichen U-förmige Form ergeben. Mit anderen Worten, die langen Schenkel der ersten L-förmigen Befestigungselemente sind im wesentlichen parallel angeordnet und die kurzen Schenkel der L-förmigen Befestigungselemente sind im wesentlichen kollinear angeordnet. Auch die langen Schenkel der zweiten L-förmigen Befestigungselemente sind parallel angeordnet und die kurzen Schenkel der L-förmigen Befestigungselemente sind im wesentlichen Grund kollinear angeordnet.

Die Erfindung betrifft auch eine radio-onkologische Patientenpositionierungsvorrichtung, die aus Carbon hergestellt ist. Der Erfinder der vorliegenden Erfindung bezeichnet die erfindungsgemäße Patientenpositionierungsvorrichtung als Offenburger Multilagerungsboard aus Carbon. Damit können sämtliche Bestrahlungstechniken auf einer Patientenpositionierungsvorrichtung ausgeführt werden. Das Board ist komplett durchstrahlfähig, da es kein Metall aufweist.

Der Anmelder behält sich vor, auf die Kopfpositioniereinrichtung separat Schutz zu beanspruchen.

### Kurze Beschreibung der Figuren

Die Erfindung wird nun unter Bezugnahme auf die beigefügten Figuren beschrieben, die exemplarische und nicht beschränkende Ausführungsformen der Erfindung zeigen, wobei
Figur 1 eine perspektivische Draufsicht auf eine radio-onkologische Patientenpositionierungsvorrichtung zeigt;
Figur 2 eine perspektivische Seitenansicht der radio-onkologischen Patientenpositionierungsvorrichtung zeigt;
Figur 3 eine weitere perspektivische Ansicht der radio-onkologischen Patientenpositionierungsvorrichtung zeigt, bei der eine
Kopfpositionierungseinrichtung detaillierter dargestellt ist;
Figur 4 eine perspektivische Ansicht einer Kopfpositionierungseinrichtung zeigt;
Figur 5 eine Seitenansicht der Kopfpositionierungseinrichtung zeigt;
Figur 6 eine perspektivische Ansicht der Kopfpositioniereinrichtung von unten darstellt; und
Figur 7 eine weitere Ausführungsform der Kopfpositionierungseinrichtung zeigt.

### Detaillierte Beschreibung der Figuren

Im Folgenden werden exemplarische Ausführungsformen der Erfindung beschrieben, um das Verständnis der Erfindung zu erleichtern. Es versteht sich, dass die Figuren nicht maßstabsgerecht sind. Es versteht sich auch, dass räumliche Angaben zu Beschreibungszwecken dienen und nicht notwendigerweise beschränkend sind.

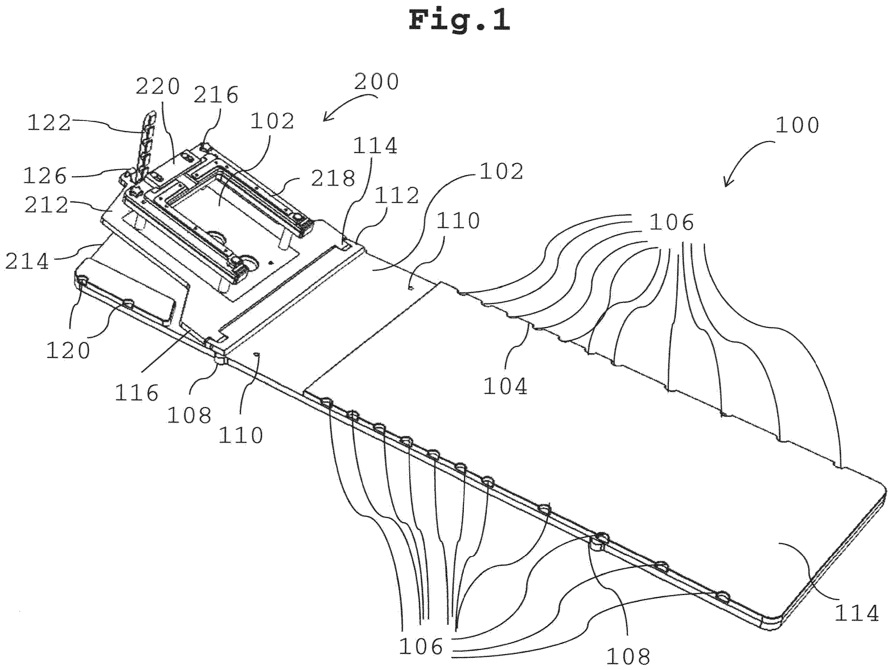

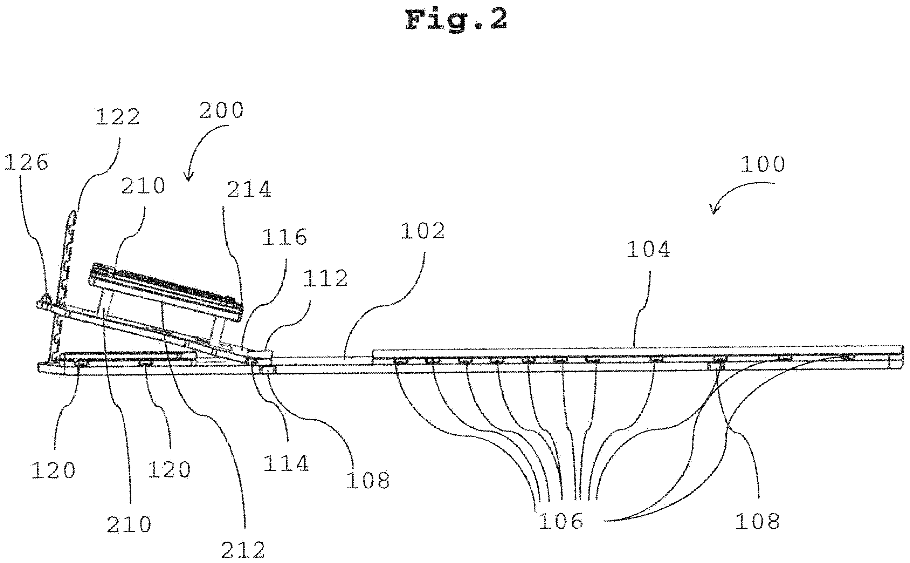

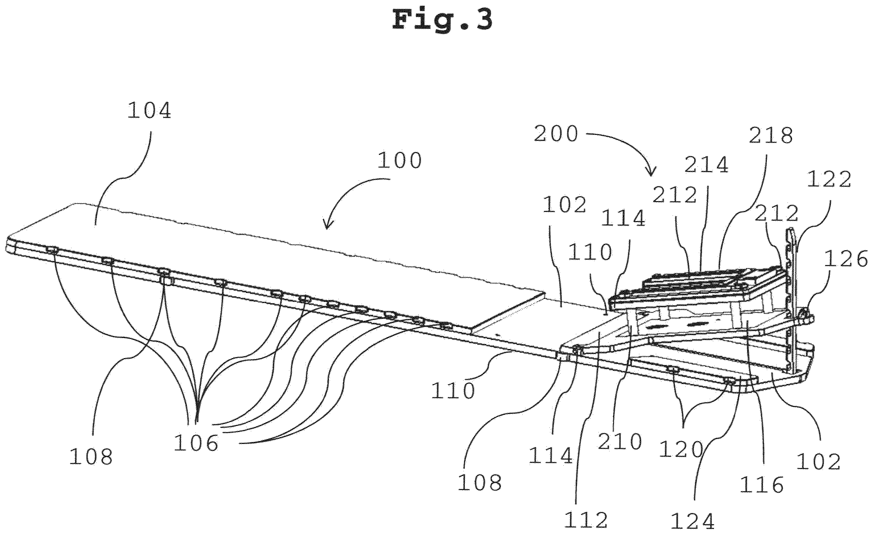

In Figuren 1, 2 und 3 ist eine erfindungsgemäße radio-onkologische Patientenpositionierungsvorrichtung (nachfolgenden Patientenpositionierungsvorrichtung) 100 mit einer erfindungsgemäßen radio-onkologischen Kopfpositioniereinrichtung (nachfolgend Kopfpositionierungseinrichtung) 200 dargestellt. Die radio-onkologische Patientenpositionierungsvorrichtung 100 und die radio-onkologische Kopfpositionierungseinrichtung 200 sind dazu ausgebildet, einen Patienten und dessen Kopf während einer radio-onkologischen Behandlung, insbesondere während eines Behandlungszyklus mit mehreren in einem vorbestimmten Zeitraum, beispielsweise mehreren Tagen, mehreren Wochen, reproduzierbar zu positionieren. Mit anderen Worten, der Patient wird auf der Patientenpositionierungseinrichtung 100 vor der Behandlung positioniert und nach der Behandlung wieder von der Patientenpositionierungsvorrichtung 100 gelöst. Für die nächste Behandlung muss der Patient wieder in der gleichen Stellung reproduzierbar auf der Patientenpositionierungseinrichtung 100 positioniert werden.

Die Patientenpositionierungsvorrichtung 100 umfasst eine Basisplatte 102, auf der eine vordere Montageplatte 104 angeordnet ist. Die Basisplatte 102 und die vordere Montageplatte sind im Wesentlichen rechteckförmig und die Montageplatte 104 ist auf der Basisplatte 102 angeordnet, die eine größere Fläche als die Montageplatte 104 aufweist. An der Basisplatte 102 sind Vorrichtungspositionierungselemente 108 ausgebildet, die dazu vorgesehen sind, die Basisplatte in einer radio-onkologischen Behandlungsvorrichtung reproduzierbar und exakt zu positionieren. Die Vorrichtungspositionierungselemente 108 erstrecken sich halbkreisförmig von der Basisplatte 102.

An der vorderen Montageplatte 104 sind eine Mehrzahl halbkreisförmiger Ausnehmungen 106 angeordnet, wobei im Einsatz an den halbkreisförmigen in Ausnehmungen 106 Positionierungssysteme zum Positionieren der Knie, der Füße, des Beckens oder dergleichen angeordnet werden können.

Die vordere Montageplatte 104 ist an dem Bereich der Basisplatte 102 angeordnet, von dem der Brustkorb, das Becken, die Beine und die Füße eines Patienten gelagert werden. Bei der in den Figuren 1 bis 3 gezeigten Ausführungsform erstreckt sich die vordere Montageplatte bzw. Rumpfstützbereich 104 nicht in den Bereich, an dem sich die Schulter und der Hals des Patienten im Einsatz befindet. An dem Bereich, an dem sich der Hals und die Schulter des Patienten befindet, sind zylinderförmige Vertiefungen 110 ausgebildet, die dazu vorgesehen sind Stützenrichtungen für die Schulter und Stützeinrichtungen für den Hals des Patienten zu halten.

Die Basisplatte 102 erstreckt sich unterhalb der Kopfpositionierungseinrichtung 200, wobei seitlich zu der Kopfpositionierungseinrichtung 200 zwei der Montageplatten 124 mit halbkreisförmige und Ausnehmungen 120 angeordnet sind.

An der Basisplatte 102 ist ein Gelenkaufnahmeblock 112 angeordnet, der über zwei Gelenke 114 gelagert. Mittels der Gelenke wird eine Kopfplatte 116 schwenkbar am Lageraufnahmeblock 112 gelagert. Die Neigung der Kopfplatte 116 kann mittels eines Rasterstabes 122, der schwenkbar auf der Basisplatte 102 angeordnet ist und unter einem vorbestimmten Winkel reproduzierbar festgelegt werden. Ein elastisches Arretierungmittel 126 verhindert, dass der Rasterstab 122 und die Kopfplatte 116 bewegt werden können.

Dadurch, dass die Kopfplatte mittels Gelenken 114 nicht lösbar (fest) mit der Basisplatte 102 schwenkbar verbunden ist, ist die Position der Kopfplatte 116 spielfrei und reproduzierbar gegenüber der Basisplatte 102 und den Vorrichtungsanbringungselementen 108 und Montageelementen 106, 120 festgelegt.

Die Basisplatte 102, die vordere Montageplatte 104, die hinteren Montageplatten 124 und/oder die Kopfplatte 116 können aus Carbon hergestellt sein. Dadurch ist die erfindungsgemäße Patientenpositionierungseinrichtung 100 einerseits besonders leicht und kann mit wenig Aufwand von medizinischem Personal gehandhabt werden. Die Ausnehmungen 106, 120 haben den Vorteil, dass weitere Positionierungseinrichtungen exakt und reproduzierbar positioniert werden können.

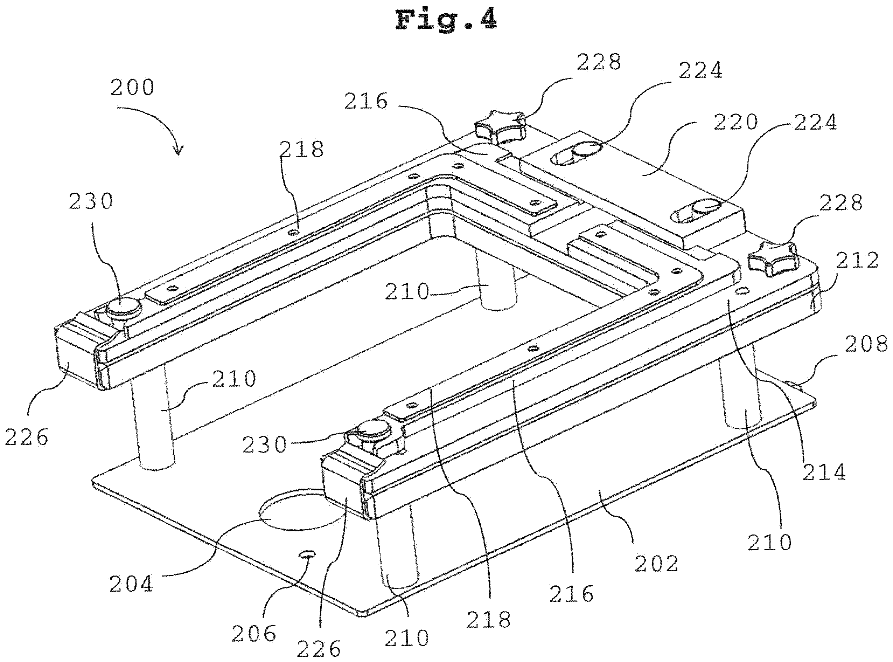

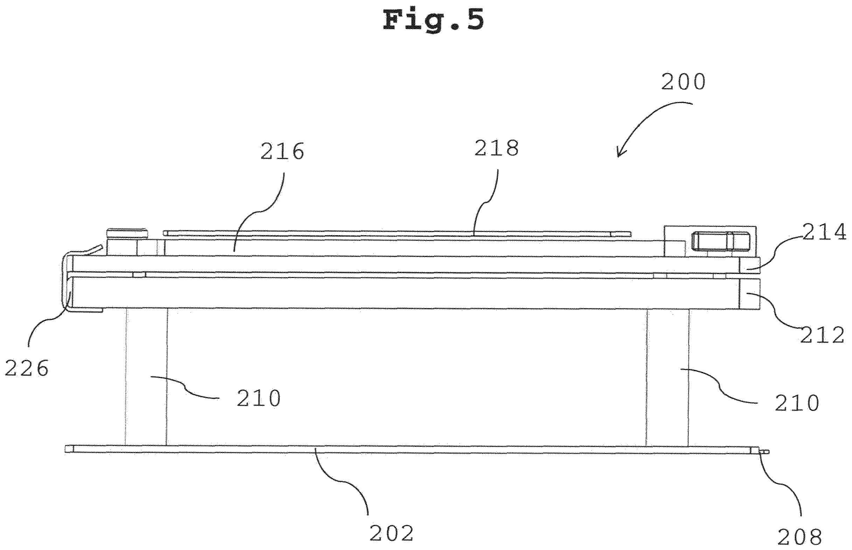

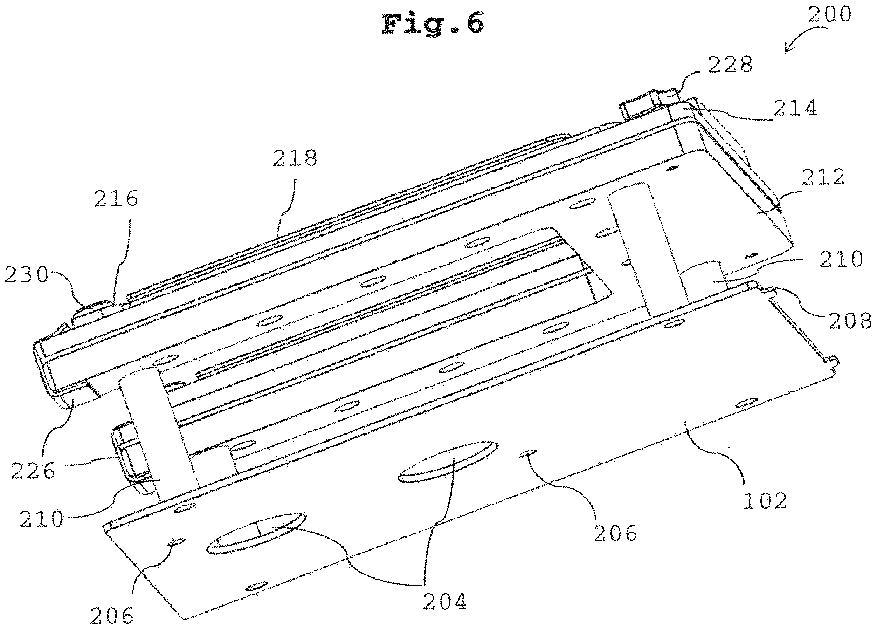

Unter Bezugnahme auf Figuren 4 bis 6 wird die erste Ausführungsform einer radio-onkologischen Kopfpositioniereinrichtung 200 (nachfolgend Kopfpositioniereinrichtung) beschrieben. Die Kopfpositioniereinrichtung 102 weist eine Grundplatte 202 mit Nasen 208 auf, die am vorderen Ende der Grundplatte angeordnet sind. Die Grundplatte weist Befestigungsöffnungen 206 auf. Die Nase 208 kann in eine komplementäre Öffnung in der Kopfplatte 106 der Patientenpositionierungsvorrichtung 100 eingesetzt werden. Anschließend wird die Grundplatte 202 in die Aufnahmeöffnung für die Grundplatte 202 geschwenkt.

Anschließend wird die Grundplatte 202 mittels Schrauben, die durch die Befestigungsöffnungen 206 der Grundplatte 202 verlaufen an der Kopfplatte 116 befestigt.

Die Grundplatte 202 weist ferner Positionierungsöffnungen 204 auf, an denen beispielsweise ein Nackenkissen angeordnet werden kann.

Bei der ersten Ausführungsform der Kopfpositioniereinrichtung 200 erstrecken sich Stützelemente 210 von der Grundplatte. Die Stützelemente 210 erstrecken sich im Einsatz neben dem Kopf eines Patienten (nicht gezeigt). An den Stützelementen 210 ist ein erster U-förmig Rahmen 212 befestigt. Der erste U-förmigen Rahmen ist in Richtung Grundplatte der Patientenpositionierungsvorrichtung 100 geöffnet. Über dem ersten U-förmigen Rahmen 212 ist ein zweiter U-förmige Rahmen 214 angeordnet, der mittels zumindest eines Drehknaufs 218 gegen den ersten U-förmigen Rahmen 212 gedrückt wird. Im Einsatz kann zwischen dem ersten U-förmigen Rahmen 212 und dem zweiten U-förmigen Rahmen 214 eine Hinterkopfmaske angeordnet sein, die im Einsatz den Hinterkopf eines Patienten stützt. Der erste U-förmigen Rahmen 212 und der zweiten U-förmigen Rahmen 214 werden durch Klammern 226 zusammengehalten.

An dem zweiten U-förmigen Rahmen 214 können zwei erste L-förmigen Befestigungselemente 216 angeordnet sein. Die zwei ersten L-förmigen Befestigungselemente 216 weisen je eine Ausnehmung 222 auf, in die ein in Längsrichtung der Kopfpositioniereinrichtung 200 bzw. der Patientenpositionierungseinrichtung 100 Klemmelement 220 geschoben werden kann. Das Klemmelement 220 kann in die Ausnehmungen 222 der ersten L-förmigen Befestigungselemente eingreifen, so dass die ersten L-förmigen Befestigungselemente 222 in Richtung zweiten U-förmigen Rahmen 214 gedrückt werden. Das Klemmelement 220 wird von Stiften 224 im zweiten U-förmigen Rahmen 214 gelagert.

Auf den ersten L-förmigen Befestigungselementen 216 sind zweite L-förmige Befestigungselemente 218 befestigt. Die zweiten L-förmigen Befestigungselemente 218 können mittels Schrauben, die durch die Öffnungen 232 der zweiten L-förmigen Befestigungselemente verlaufen, an den ersten L-förmigen Befestigungselementen 216 befestigt werden. Zwischen dem ersten L-förmigen Befestigungselementen 216 und den zweiten L-förmigen Befestigungselementen 218 kann eine Gesichtsmaske geklemmt werden. Vor dem Anordnen der Gesichtsmaske wird die Maske mittels eines Wassersbades mit einer Temperatur von etwa 65°C erwärmt.

Bei der in den Figuren 4 bis 6 gezeigten Ausführungsform sind der erste U-förmige Rahmen 212 und der zweite U-förmigen Rahmen 214 in Längsrichtung der Kopfpositionierungseinrichtung 200 bzw. der Patientenpositionierungsvorrichtung 100 geöffnet. Die langen Schenkel der zwei ersten L-förmigen Befestigungselemente 216 und die langen Schenkel der zwei zweiten L-förmigen Befestigungselemente 218 sind auch in Längsrichtung der Kopfpositionierungseinrichtung 200 bzw. der Patientenpositionierungseinrichtung 100 gerichtet.

Die Kopfpositionierungseinrichtung 200 kann aus Carbon hergestellt sein.

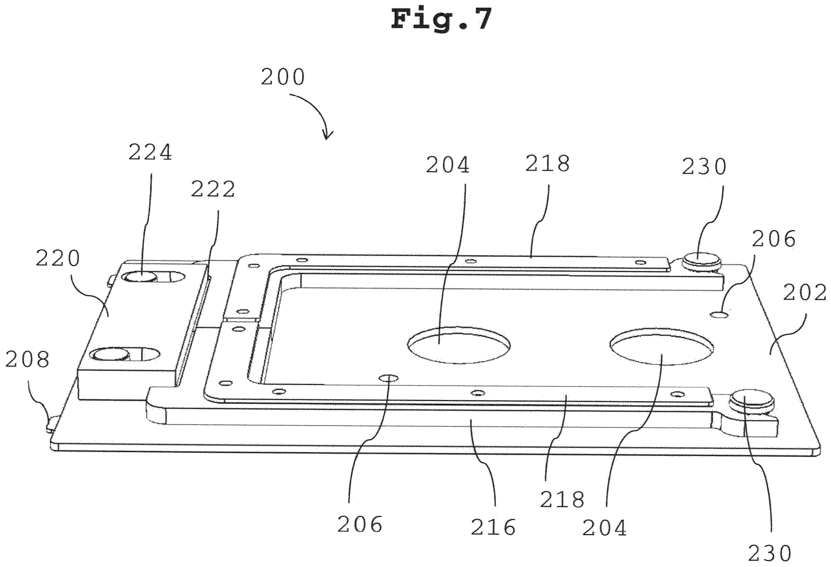

Figur 7 zeigt eine zweite Ausführungsform der erfindungsgemäßen Positionierungseinrichtung 200', die im wesentlichen der ersten Ausführungsform 200 entspricht, die zuvor unter Bezugnahme auf Figuren 4 bis 6 beschrieben wurden. Im Sinne der Prägnanz werden folglich nur die Unterschiede zwischen der zweiten Ausführungsform 200' und der ersten Ausführungsform 200 der erfindungsgemäßen Kopfpositionierungseinrichtung beschrieben.

Die ersten L-förmigen Befestigungselemente 218 sind mittels eines Anschlages 230 und eines Klemmelementes 222, deren Funktion demjenigen entspricht, die unter Bezugnahme auf Figuren 4 bis 6 hinsichtlich der ersten Ausführungsform 200 beschrieben wurden, an der Grundplatte 202 befestigt. Zwischen den ersten förmigen Befestigungselementen 216 und den zweiten L-förmigen Befestigungselemente 218 kann eine Gesichtsmaske angeordnet sein, die auch als Vorderkopfmaske bezeichnet wird. Der Kopf des Patienten kann auf einem Kissen ruhen, das an den Anbringungsöffnungen 204 angebracht ist.

Die Grundplatte 202 wird mittels der Nasen 208 in eine Öffnung in der Kopfplatte 116 der Patientenpositionierungsvorrichtung 100 spielfrei angeordnet und mittels Schrauben, die durch die Befestigungsöffnungen 206 der Grundplatte 202 verlaufen, spielfrei an der Kopfleiste 116 angeordnet.

Auch bei dieser Ausführungsform verlaufen die langen Schenkel der beiden ersten L-förmigen Befestigungseinrichtungen 216 und der zweiten L-förmigen Befestigungseinrichtungen 218 in Längsrichtung der Kopfpositioniereinrichtung 200' bzw. Patientenpositionierungsvorrichtung 200'.

Die zweite Ausführungsform der Kopfpositionierungseinrichtung 200' kann aus Carbon hergestellt sein.

Die vorliegende Erfindung hat den Vorteil, dass mittels der Kopfpositioniereinrichtung 200, 200' der Kopf eines Patienten reproduzierbar gelagert werden kann. Die Kopfpositioniereinrichtung 200, 200' kann von einem medizinischen Personal flexibel an die Bedürfnisse eines Patienten angepasst werden. Ferner kann die erfindungsgemäße Kopfpositioniereinrichtung 200, 200' in kurzer Zeit einsatzfähig gemacht werden, was insbesondere bei mehrfachen Behandlungen relevant ist.

Die Kopfpositioniereinrichtung 200, 200' und die Patientenpositionierungseinrichtung 100 sowie die daran angebrachten Stützeinrichtungen (nicht gezeigt) werden bei Beginn einer Behandlungsperiode auf den Patienten angepasst und werden während der Behandlungsperiode nicht verändert. Dadurch kann zum einen die Genauigkeit der Behandlung über die gesamte Behandlungsperiode erhöht werden und zum anderen die Rüstzeit bei jeder Bestrahlung innerhalb der Behandlungsperiode reduziert werden, sowie das Personal entlastet werden.

Ferner weist die erfindungsgemäße Patientenpositionierungseinrichtung den Vorteil auf, dass die Kopfpositioniereinrichtung mittels der Gelenke 114 der Kopfplatte 216 spielfrei und reproduzierbar gegenüber der Basisplatte 102 der Patientenpositionierungseinrichtung 100 angeordnet werden kann, wodurch die Qualität der medizinischen Behandlung erhöht wird.

Im Schädelbereich können sowohl Vorderkopfmasken bei der Ausgestaltung der Kopfpositioniereinrichtung 200' gemäß Fig. 7 sowie Ganzschädelmasken bei der Ausgestaltung der Kopfpositioniereinrichtung 200 gemäß Fig. 4 angefertigt werden.

Für die Anbringung der Vorderkopfmaske bei der Kopfpositioniereinrichtung 200' gemäß Figur 7 wird ein Maskenmaterial auf den ersten L-förmigen Befestigungselementen 216 aufgeschraubtem durch die zweiten L-förmigen Befestigungselementen 218 mittels Kunststoffschrauben befestigt. Das Maskenmaterial wird mittels einem etwa 65°C warmen Wasserbad erwärmt und über die Schädelvorderseite des Patienten gelegt und angeformt. Nach ca. 1 Minute ist das Maskenmaterial ausgehärtet. Ersten L-förmigen Befestigungselemente 216 wird dabei an zwei Pilzköpfen (Anschlägen) 230 sowie einem Spannblock (Klemmelement) 220 präzise auf der Grundplatte 202 positioniert. Der Hinterkopf liegt dabei auf einem Nackenkissen, welches mittels der Befestigungsöffnung 204 fixiert wird.

Bei Ganzschädelmasken wird der Hinterkopf freitragend mittels der Kopfpositioniereinrichtung 200 gemäß Figur 4 positioniert. Die Grundplatte 202, die Stützelemente 210 und der erste U-förmige Rahmen 212 bilden eine feste Einheit, die immer in diesem Zustand bleibt. Der erste U-förmige Rahmen 212und der zweite U-förmige Rahmen 214 bilden eine Sandwichhalterung, in die das vorgewärmte Maskenmaterial eingespannt und individuell an den Hinterkopf eines Patienten angepasst wird. Der zweite U-förmige Rahmen 216 wird mittels des Drehknaufs 228 und der Klammern 226 am ersten U-förmigen Rahmen befestigt. Positionsstifte am erstem U-förmigen Rahmen 212 und komplementäre Öffnungen 227 am zweiten U-förmigen Rahmen 214 ermöglichen eine reproduzierbare Positionierung der beiden U-förmigen Rahmen zueinander.

Anschließend wird die Vorderkopfmaske angefertigt. Dies erfolgt direkt im Anschluss an die Herstellung der Hinterkopfmaske, während der Patient dann in der Hinterkopfmaske liegt. Für die Vorderkopfmaske wird ein Maskenmaterial auf den ersten L-förmigen Befestigungselementen 216 angeordnet und durch die zweiten L-förmigen Befestigungselemente 218 mittels Kunststoffschrauben befestigt. Das Maskenmaterial wird mittels eines etwa 65°C warmen Wasserbades erwärmt und über die Schädelvorderseite des Patienten gelegt und angeformt. Nach ca. 1 Minute ist das Maskenmaterial ausgehärtet. Die ersten L-förmigen Befestigungselemente 216 werden dann am zweiten U-förmigen Rahmen 214 befestigt. Die ersten L-förmigen Befestigungselemente 216 werden dabei an zwei Pilzköpfen (Anschläge) 230 sowie einem Spannblock (Klemmelement) 220 präzise auf der Kopfpositioniereinrichtung 200 bzw. dem zweiten U-förmigen Rahmen 214 positioniert.

Beide Schädelmasken können zusammen oder individuell auch einzeln verwendet werden.

Zusätzlich können Schultermaskeneinrichtung zur genauen Fixierung von Schädel, Hals und Schulter angewendet werden. Die Schultermaskeneinrichtung werden in die Basisplatte 102 (Aussparung 102) eingelegt und durch Stifte in den Vertiefungen 110 fixiert.

Anschließend wird die Maske nach dem gleichen Prinzip wie die Schädelmasken auch zusätzlich über die Schultern positioniert.

Die Grundplatte 102 kann präzise und reproduzierbar auf der Tischplatte des Bestrahlungsgerätes positioniert werden kann. Durch einen Rasterstab 122 können unterschiedliche Winkel zur Schädelneigung eingestellt werden, indem das Neigungsteil 116 über Scharniere 114 in die Ausnehmungen des Rasterstabes 122 gehalten wird.

Über die Positionierungselemente 106 an der Montageplatte 104 können weitere Halterungen auch von anderen Firmen auf der Patientenpositionierungsvorrichtung 100 angeordnet werden. Mittels dieser Halterungen werden u.a. die Beine über eine handelsübliche Beinhalterung in der jeweiligen Patientenlagerung positioniert.

Die vorliegende Erfindung betrifft eine aus Carbon hergestellten radio-onkologischen Patientenpositionierungsvorrichtung, die erstmals eine Kopfpositionierungseinrichtung, die den Einsatz einer hochpräzisen, für stereotaktische Strahlentherapie geeigneten Doppelmaske (Hinterkopf und Gesicht) erlaubt, mit einer kippbaren, fest an der Positionierungseinrichtung fixierten Platte kombiniert.

Dies ergibt für die tägliche Praxis der Strahlentherapie erhebliche Vorteile. Bestimmte Lagerungspositionen des Patienten, welche für die Schonung von empfindlichem Gewebe bei gleichzeitiger Applikation einer hohen Strahlendosis im Tumor vorteilhaft sind, können durch die Erfindung in der notwendigen Präzision überhaupt erst erreicht werden. Die Verwendung von Carbon erlaubt es, sämtliche Teile der Vorrichtung ohne wesentliche Schwächung der Dosis zu durchstrahlen bei gleichzeitiger höchster Präzision der Lagerung. Das der Erfindung zu Grunde liegende Konzept der Integration mehrerer Lagerungskonstruktionen verhindert ein Umbauen des Bestrahlungstisches für die einzelnen Patienten. Dies erhöht die Präzision, da mit jedem Aufbau einer neuen Lagerungsvorrichtung Fehler einhergehen, die mit den Anforderungen an hochpräzise Bestrahlungen nicht vereinbar sind. Weiterhin erleichtert die Erfindung die tägliche Arbeit des Personals in der Strahlentherapie. Die exakte Reproduzierbarkeit der Patientenposition wird durch die Erfindung verbessert (erstmals Kombination einer reproduzierbar hochpräzisen, zweiteiligen Maske mit einem kippbaren Lagerungsbrett). Die Erfindung integriert sehr einfach zu handhabende Fixierungsmöglichkeiten (Mechanismen zur Einrastung von Masken und anderen Lagerungshilfen). Diese Vorrichtungen erlauben ein zügiges Anfertigen der Maske, so dass Patienten bei diesem Vorgang wesentlich verkürzte Liegezeiten haben, was für Schwerstkranke mit Hirntumoren, die in derartigen Masken gelagert werden, ein großer Vorteil ist. Dieselben Vorrichtungen für das exakte Einrasten und Befestigen der Präzisionsmaske ermöglichen beim täglichen Reproduzieren der Patientenlagerung ebenso eine erhebliche Zeitersparnis. Die Erfindung erlaubt die Kombination verschiedener Bauteile zur Patientenlagerung, beispielsweise Brett bzw. Platte für die Schulterlagerung und hochpräzise Lagerungsmaske. Dies ist mit bisherigen Lagerungsvorrichtungen so flexibel nicht möglich.

## Patentansprüche

1. Radio-onkologische Patientenpositionierungsvorrichtung (100), aufweisend
- einen Rumpfstützbereich (102, 104), der dazu ausgebildet ist, den Rumpf eines Patienten zu stützen, mit
- ersten Positionierungselementen (106), die dazu eingerichtet sind
- eine Kniepositionierungseinrichtung und/oder
- eine Beckenpositionierungseinrichtung
lösbar anzuordnen,
- einen Schulterstützbereich (102), der zweite Positionierungselemente (110) aufweist, die dazu eingerichtet sind,
- eine Halspositionierungseinrichtung; und/oder
- eine Schulterpositionierungseinrichtung
lösbar anzuordnen; und
- einen Kopfstützbereich (116);
**dadurch gekennzeichnet, dass**
der Kopfstützbereich (116) mittels zumindest eines Gelenks (114) schwenkbar am Schulterstützbereich (102) oder an einer Basisplatte (102) angeordnet ist, auf der der Rumpfstützbereich (104) angeordnet ist, angeordnet ist; und
- wobei der Kopfstützbereich (116) so eingerichtet ist, dass eine radio-onkologische Kopfpositionierungseinrichtung (200; 200') lösbar am Kopfstützbereich (116) anbringbar ist, wobei an der Kopfpositionierungseinrichtung (200; 200') eine Hinterkopfmaske und/oder eine Gesichtsmaske anordenbar ist.

2. Radio-onkologische Patientenpositionierungsvorrichtung (100) nach Anspruch 1, wobei der Kopfstützbereich (116) eine Arretierungsvorrichtung (122, 126) aufweist, die ermöglicht, den Kopfstützbereich (106) unter unterschiedlichen Winkelstellungen gegenüber dem Rumpfstützbereich (102, 104) einstellbar abzustützen.

3. Radio-onkologische Patientenpositionierungsvorrichtung (100) nach Anspruch 1 oder 2, wobei sich die Basisplatte (102) zumindest teilweise unter den Rumpfstützbereich (104) und unter den Kopfstützbereich (116) erstreckt.

4. Radio-onkologische Patientenpositionierungsvorrichtung (100) nach einem der Ansprüche 1 bis 4, ferner aufweisend eine radio-onkologische Kopfpositionierungseinrichtung (200), die zum Halten des Kopfes eines Patienten während einer radio-onkologischen Behandlung ausgebildet ist; aufweisend:
- eine Grundplatte (202), die zum Koppeln mit einer radio-onkologischen Patientenpositionierungsvorrichtung ausgebildet ist;
- zumindest ein Stützelement (210), das sich von der Grundplatte (202) erstreckt;
- einen ersten U-förmigen Rahmen (212), der an dem zumindest einem Stützelement (210) angeordnet ist;
- einen zweiten U-förmigen Rahmen (214), der lösbar an dem ersten U-förmigen Rahmen (212) angeordnet ist; und
- zwei erste L-förmige Befestigungselemente (216), die am zweiten U-förmigen Rahmen (214) angeordnet sind.

5. Radio-onkologische Patientenpositionierungsvorrichtung (100) nach einem der Ansprüche 1 bis 4 **gekennzeichnet durch** zwei zweite L-förmige Befestigungselemente (216), die an den zwei ersten L-förmigen Befestigungselementen (218) lösbar angeordnet sind.

6. Radio-onkologische Patientenpositionierungsvorrichtung (100) nach einem der Ansprüche 1 bis 5, **gekennzeichnet durch** ein in Längsrichtung des ersten U-förmigen Rahmens (212) und des zweiten U-förmigen Rahmens (214) verschiebbaren Klemmelement (220), das in seiner geschlossenen Stellung die zwei ersten L-förmigen Befestigungselemente (216) gegen den zweiten U-förmigen Rahmen (214) drückt und in seiner offenen Stellung in Längsrichtung vom offenen Ende des ersten U-förmigen Rahmens (212) und des zweiten U-förmigen Rahmens (214) betrachtet hinter den zwei ersten L-förmigen Befestigungselementen (216) angeordnet ist.

7. Radio-onkologische Patientenpositionierungsvorrichtung (100) nach einem der Ansprüche 1 bis 6, **dadurch gekennzeichnet, dass** zwischen dem ersten U-förmigen Rahmen (212) und dem zweiten U-förmigen Rahmen (214) die Hinterkopfmaske anordenbar ist.

8. Radio-onkologische Patientenpositionierungsvorrichtung (100) nach Anspruch 6 oder 7, **dadurch gekennzeichnet, dass** zwischen den ersten L-förmigen Befestigungselementen (216) und den zweiten L-förmigen Befestigungselementen (218) die Gesichtsmaske anordenbar ist.

9. Radio-onkologische Patientenpositionierungsvorrichtung (100) nach Anspruch einem der Ansprüche 1 bis 3, ferner aufweisend eine radio-onkologische Kopfpositionierungseinrichtung (200'), die zum Halten des Kopfes eines Patienten während einer radio-onkologischen Behandlung ausgebildet ist; aufweisend:
- eine Grundplatte (202), die zum Koppeln mit einer radio-onkologischen Patientenpositionierungsvorrichtung ausgebildet ist;
- zwei erste L-förmige Befestigungselemente (216), die an der Grundplatte angeordnet sind; und
- zwei zweite L-förmige Befestigungselemente (218), die an den ersten L-förmigen Befestigungselementen lösbar angeordnet sind;
- wobei zwischen den ersten L-förmigen Befestigungselementen (216) und den zweiten L-förmigen Befestigungselementen (218) die Gesichtsmaske anordenbar ist.

10. Radio-onkologische Patientenpositionierungsvorrichtung (100) nach Anspruch 9, **gekennzeichnet durch** ein in Längsrichtung der radio-onkologischen Kopfpositionierungseinrichtung (200') verschiebbaren Klemmelement (220), das in seiner geschlossenen Stellung die zwei ersten L-förmigen Befestigungselemente (216) gegen die Grundplatte (202) drückt und in seiner offenen Stellung in Längsrichtung vom offenen Ende der zwei ersten L-förmigen Befestigungselemente (216) betrachtet hinter den zwei ersten L-förmigen Befestigungselementen (218) angeordnet ist.

## Claims

1. A radio-oncological patient positioning device (100), comprising
- a torso support region (102, 104) adapted to support the torso of a patient, comprising
- first positioning members (106) adapted to releasably arrange
- a knee positioning means and/or
- a pelvic positioning device;
- a shoulder support portion (102) having second positioning members (110) adapted to releasably arrange
- a neck positioning means; and/or
- a shoulder positioning device; and
- a head support portion (116);
**characterized in that**:
the head support portion (116) is pivotally disposed, by means of at least one joint (114), on the shoulder support portion (102) or on a base plate (102) on which the torso support portion (104) is disposed; and
- wherein the head support portion (116) is adapted such that a radio-oncological head positioning device (200; 200') is detachably attachable to the head support portion (116), wherein an occipital mask and/or a face mask is arrangeable on the head positioning device (200; 200').

2. The radio-oncological patient positioning device (100) according to claim 1, wherein the head support portion (116) comprises a locking device (122, 126), which enables the head support portion (106) to be adjustably supported at different angular positions relative to the torso support portion (102, 104).

3. The radio-oncology patient positioning device (100) according to claim 1 or 2, wherein the base plate (102) extends at least partially below the torso support region (104) and below the head support region (116).

4. The radio-oncology patient positioning device (100) according to any one of claims 1 to 4, further comprising a radio-oncology head positioning device (200) adapted to support a patient's head during radio-oncology treatment; comprising:
- a base plate (202) adapted to couple to a radio-oncology patient positioning device;
- at least one support member (210) extending from the base plate (202); and
- a first U-shaped frame (212) disposed on the at least one support member (210); and
- a second U-shaped frame (214) removably disposed on the first U-shaped frame (212); and
- two first L-shaped attachment members (216) disposed on the second U-shaped frame (214).

5. The radio-oncology patient positioning device (100) according to any one of claims 1 to 4, **characterized by** two second L-shaped attachment members (216) removably disposed on the two first L-shaped attachment members (218).

6. The radio-oncology patient positioning device (100) according to any one of claims 1 to 5, **characterized by** a clamping member (220) slidable in the longitudinal direction of the first U-shaped frame (212) and the second U-shaped frame (214), which in its closed position presses the two first L-shaped fastening elements (216) against the second U-shaped frame (214) and in its open position is arranged behind the two first L-shaped fastening elements (216) as viewed in the longitudinal direction from the open end of the first U-shaped frame (212) and the second U-shaped frame (214).

7. The radio-oncological patient positioning device (100) according to any one of claims 1 to 6, **characterized in that** between the first U-shaped frame (212) and the second U-shaped frame (214) the occipital mask is arrangeable.

8. The radio-oncological patient positioning device (100) according to claim 6 or 7, **characterized in that** between the first L-shaped mounting elements (216) and the second L-shaped mounting elements (218) the face mask is arrangeable.

9. The radio-oncological patient positioning device (100) according to any one of claims 1 to 3, further comprising a radio-oncological head positioning device (200') adapted to hold a patient's head during a radio-oncological treatment; comprising:
- a base plate (202) adapted to be coupled to a radio-oncology patient positioning device; and
- two first L-shaped fasteners (216) disposed on the base plate; and
- two second L-shaped fastening members (218) releasably disposed on the first L-shaped fastening members;
- wherein the facemask is disposable between the first L-shaped mounting members (216) and the second L-shaped mounting members (218).

10. The radio-oncological patient positioning device (100) according to claim 9, **characterized by** a clamping element (220) displaceable in the longitudinal direction of the radio-oncological head positioning device (200'), which in its closed position presses the two first L-shaped fastening elements (216) against the base plate (202) and in its open position is arranged behind the two first L-shaped fastening elements (218) as viewed in the longitudinal direction from the open end of the two first L-shaped fastening elements (216).

## Revendications

1. Dispositif de positionnement de patient de radio-oncologie (100) ; comprenant :
- une région de support de torse (102, 104) qui est adaptée pour supporter le torse d'un patient, comprenant :
- des premiers éléments de positionnement (106) qui sont adaptés pour agencer de façon libérable :
- un moyen de positionnement de(s) genou(x) ; et/ou
- un dispositif de positionnement de bassin ;
- une partie de support d'épaule(s) (102) qui comporte des seconds éléments de positionnement (110) qui sont adaptés pour agencer de façon libérable :
- un moyen de positionnement de cou ; et/ou
- un dispositif de positionnement d'épaule(s) ; et
- une partie de support de tête (116) ; **caractérisé en ce que** :
la partie de support de tête (116) est disposée de façon pivotante, au moyen d'au moins une articulation (114), sur la partie de support d'épaule(s) (102) ou sur une plaque de base (102) sur laquelle la partie de support de torse (104) est disposée ; et
- dans lequel la partie de support de tête (116) est adaptée de telle sorte qu'un dispositif de positionnement de tête de radio-oncologie (200 ; 200') puisse être lié de façon détachable à la partie de support de tête (116), dans lequel un masque occipital et/ou un masque de visage peut être agencé sur le dispositif de positionnement de tête (200 ; 200').

2. Dispositif de positionnement de patient de radio-oncologie (100) selon la revendication 1, dans lequel la partie de support de tête (116) comprend un dispositif de blocage (122, 126), lequel dispositif de blocage permet que la partie de support de tête (106) soit supportée de façon réglable selon différentes positions angulaires par rapport à la partie de support de torse (102, 104).

3. Dispositif de positionnement de patient de radio-oncologie (100) selon la revendication 1 ou 2, dans lequel la plaque de base (102) est étendue au moins partiellement en dessous de la région de support de torse (104) et en dessous de la région de support de tête (116).

4. Dispositif de positionnement de patient de radio-oncologie (100) selon l'une quelconque des revendications 1 à 4, comprenant en outre un dispositif de positionnement de tête de radio-oncologie (200) qui est adapté pour supporter la tête d'un patient pendant un traitement de radio-oncologie ; comprenant :
- une plaque de base (202) qui est adaptée pour être couplée à un dispositif de positionnement de patient de radio-oncologie ;
- au moins un élément de support (210) qui est étendu depuis la plaque de base (202) ; et
- une première structure en forme de U (212) qui est disposée sur l'au moins un élément de support (210) ; et
- une seconde structure en forme de U (214) qui est disposée de façon amovible sur la première structure en forme de U (212) ; et
- deux premiers éléments de liaison en forme de L (216) qui sont disposés sur la seconde structure en forme de U (214).

5. Dispositif de positionnement de patient de radio-oncologie (100) selon l'une quelconque des revendications 1 à 4, **caractérisé par** deux seconds éléments de liaison en forme de L (216) qui sont disposés de façon amovible sur les deux premiers éléments de liaison en forme de L (218).

6. Dispositif de positionnement de patient de radio-oncologie (100) selon l'une quelconque des revendications 1 à 5, **caractérisé par** un élément de fixation par serrage (220) qui peut coulisser dans la direction longitudinale de la première structure en forme de U (212) et de la seconde structure en forme de U (214), lequel élément de fixation par serrage, dans sa position fermée, presse les deux premiers éléments de liaison ou de fixation en forme de L (216) contre la seconde structure en forme de U (214) et dans sa position ouverte, est agencé derrière les deux premiers éléments de liaison ou de fixation en forme de L (216), tel que vu dans la direction longitudinale depuis l'extrémité ouverte de la première structure en forme de U (212) et de la seconde structure en forme de U (214).

7. Dispositif de positionnement de patient de radio-oncologie (100) selon l'une quelconque des revendications 1 à 6, **caractérisé en ce que**, entre la première structure en forme de U (212) et la seconde structure en forme de U (214), le masque occipital peut être agencé.

8. Dispositif de positionnement de patient de radio-oncologie (100) selon la revendication 6 ou 7, **caractérisé en ce que**, entre les premiers éléments de liaison ou de montage en forme de L (216) et les seconds éléments de liaison ou de montage en forme de L (218), le masque de visage peut être agencé.

9. Dispositif de positionnement de patient de radio-oncologie (100) selon l'une quelconque des revendications 1 à 3, comprenant en outre un dispositif de positionnement de tête de radio-oncologie (200') qui est adapté pour maintenir la tête d'un patient pendant un traitement de radio-oncologie ; comprenant :
- une plaque de base (202) qui est adaptée pour être couplée à un dispositif de positionnement de patient de radio-oncologie ; et
- deux premiers moyens ou éléments de liaison ou de fixation en forme de L (216) qui sont disposés sur la plaque de base ; et
- deux seconds moyens ou éléments de liaison ou de fixation en forme de L (218) qui sont disposés de façon libérable sur les deux premiers moyens ou éléments de liaison ou de fixation en forme de L ;
- dans lequel le masque de visage peut être disposé entre les premiers moyens ou éléments de liaison ou de montage en forme de L (216) et les seconds moyens ou éléments de liaison ou de montage en forme de L (218).

10. Dispositif de positionnement de patient de radio oncologie (100) selon la revendication 9, **caractérisé par** un élément de fixation par serrage (220) qui peut être déplacé dans la direction longitudinale du dispositif de positionnement de tête de radio-oncologie (200'), lequel élément de fixation par serrage, dans sa position fermée, presse des deux premiers moyens ou éléments de liaison ou de fixation en forme de L (216) contre la plaque de base (202) et dans sa position ouverte, est agencé derrière les deux premiers moyens ou éléments de liaison ou de fixation en forme de L (218), tel que vu dans la direction longitudinale depuis l'extrémité ouverte des deux premiers moyens ou éléments de liaison ou de fixation en forme de L (216).
